# EUROPÄISCHE PATENTSCHRIFT

(11) **EP 1 725 675 B1**
(45) Veröffentlichungstag und Bekanntmachung des Hinweises auf die Patenterteilung: **26.06.2013**
(21) Anmeldenummer: 05715977.4
(22) Anmeldetag: 11.03.2005
(51) Int. Cl.: C12Q 1/04, C12Q 1/18

(54) **VERFAHREN ZUR ERMITTLUNG VON GEGEN GERUCHSKEIME WIRKSAMEN SUBSTANZEN, DIE IM AXILLAREN BEREICH PRÄBIOTISCH WIRKSAM SIND**
METHOD FOR DETERMINING SUBSTANCES WITH A PRE-BIOTIC ACTION IN THE AXILLARY AREA AGAINST BODY ODOR GERMS
PROCEDE POUR DETERMINER DES SUBSTANCES QUI ONT UNE ACTION PREBIOTIQUE DANS LA ZONE AXILLARE SUR DES GERMES RESPONSABLES DE L'APPARITION DE MAUVAISES ODEURS

(30) Priorität: 18.03.2004 DE 102004013697
(43) Veröffentlichungstag der Anmeldung: 29.11.2006
(73) Patentinhaber: Henkel AG & Co. KGaA, 40589 Düsseldorf (DE)
(72) Erfinder: BOCKMÜHL, Dirk, 42113 Wuppertal (DE); HÖHNE, Heide-Marie, 50189 Elsdorf (DE); JASSOY, Claudia, 40237 Düsseldorf (DE); SCHOLTYSSEK, Regine, 40822 Mettmann (DE); BANOWSKI, Bernhard, 40597 Düsseldorf (DE); WADLE, Armin, 40699 Erkrath (DE); SÄTTLER, Andrea, 40225 Düsseldorf (DE); NIEVELER, Silke, 41236 Mönchengladbach (DE); BREVES, Roland, 40822 Mettmann (DE)
(86) Internationale Anmeldenummer: PCT/EP2005/002616
(87) Internationale Veröffentlichungsnummer: WO 2005/093084

(56) Entgegenhaltungen:
- EP-A- 0 091 745
- EP-A- 1 181 866
- EP-A- 1 184 030
- DE-A1- 10 022 616
- US-A- 5 837 482
- US-A1- 2003 195 263

## Beschreibung

Die vorliegende Erfindung betrifft ein Verfahren zur Identifizierung von Substanzen, die gegen Geruchskeime auf der Haut und insbesondere im axillaren Bereich präbiotisch wirksam sind .

Körpergeruch entsteht durch den Abbau von Schweißbestandteilen durch Bakterien der Hautflora, insbesondere im axillaren Bereich. Die beteiligten Arten werden üblicherweise über die Kultivierung von Achselproben auf Bakteriennährböden ermittelt. Diese Methode hat jedoch den Nachteil, dass nicht kultivierbare Arten, die an der Entstehung von Körpergeruch beteiligt sind, nicht erfasst werden. Außerdem lassen sich einige Bakterienarten besser kultivieren als andere, so dass die Ergebnisse keine Aussage bezüglich der Bedeutung der identifizierten Bakterien mit Hinblick auf die Entstehung von Körpergeruch zulassen. Folglich fehlt auch die Voraussetzung für Versuche zur effizienten Bewertung von Deodorantien. Infolgedessen kommt es in Deodorantien zum Einsatz antibakterieller Substanzen, die aufgrund des falschen Wirkspektrums nur eine unzureichende Deoleistung besitzen.

D. Taylor et al., Int. J. of Cosm. Science, 2003, 25, 137-145, beschreibt ein Verfahren zur Charakterisierung der Hautmikroflora aus dem menschlichen Achselbereich, das durch die Entnahme, Kultivierung und 16S rRNA-Analyse der geruchsassoziierten Kime gekennzeichnet ist.

Aufgabe der vorliegenden Erfindung war es daher ein Verfahren zur Verfügung zu stellen, das es erlaubt, in Bezug auf geruchsbildende Keime präbiotisch wirksame Substanzen zur Verfügung zu stellen.

Ein Teil der Aufgabe bestand hierbei insbesondere darin, ein Verfahren zur Verfügung zu stellen, das es erlaubt, die für die Geruchsbildung verantwortlichen Keime zu ermitteln bzw. Unterschiede im Mikrofloraprofil zwischen Probanden mit starkem und/oder unangenehmen Körpergeruch und Probanden mit schwachem Körpergeruch festzustellen, um hierdurch die Voraussetzung zur Ermittlung gegen Geruchskeime präbiotisch wirksamer Substanzen zu schaffen.

Gelöst wurde diese Aufgabe durch molekularbiologische Methoden, mit denen man die Zusammensetzung von Proben analysieren kann und hierbei insbesondere auch schlecht kultivierbare und/oder nicht kultivierbare Mikroorganismen nachweisen kann. Vor allem ermöglicht es die Methode, quantitative Aussagen über die Zusammensetzung von Proben zu treffen, so dass ermittelt werden kann, welche Mikroorganismen bei Körpergeruch vermehrt auftreten bzw. welche Unterschiede im Mikrofloraprofil zwischen Personen mit starkem und/oder unangenehmen Körpergeruch und Personen mit schwachem und/oder angenehmen Körpergeruch bestehen.

Die durch die molekularbiologischen Methoden ermittelten Keime können nun gezielt eingesetzt werden, um Substanzen zu ermitteln, die in Bezug auf Körpergeruch präbiotisch wirksam sind, indem sie hemmend auf das Wachstum der geruchsbildenden Keime wirken und/oder förderlich auf das Wachstum der geruchsneutralen Keime wirken. Die durch die molekularbiologischen Methoden ermittelten Keime können des weiteren eingesetzt werden, um bereits bekannte Deodorant-Wirkstoffe auf ihre Wirksamkeit hin zu überprüfen.

Desweiteren wind beschrieben, daβ durch die genannten molekularbiologischen Methoden festgestellt werden konnte dass Probanden mit starkem Körpergeruch eine andere Zusammensetzung der bakteriellen Mikroflora aufweisen als Menschen mit schwachem Körpergeruch.

Insbesondere waren folgende Merkmale für Personen mit starkem Körpergeruch im Vergleich zu Personen mit schwachem Körpergeruch typisch:
a) verminderter Anteil an *Staphylococcus epidermidis*
*b)* erhöhter Anteil an *Staphylococcus hominis*
c) leicht erhöhter Anteil an *Anaerococcus octavius*
d) leicht erhöhter Anteil an bestimmten *Corynebacterium Spezies*

Die ermittelten Keime konnten nun, auch in Verbindung mit bereits bekannten Verursachern von Achselgeruch (*Micrococcus luteus, Corynebacterium CDC G2*), zur Bewertung der Deoleistung von Wirkstoffen herangezogen werden. Dabei konnte erstmals nicht nur die hemmende Wirkung von Substanzen berücksichtigt werden, sondern auch eine fördernde Wirkung auf *S. epidermidis* mit in die Bewertung einbezogen werden.

Ein Gegenstand der vorliegenden Erfindung ist daher ein Verfahren zur Ermittlung von Keimen, die für die Geruchsbildung und/oder Geruchshemmung verantwortlich sind, bestehend aus folgenden Schritten:
a) Entnahme von Vergleichsproben von Probanden mit starkem und schwachem Körpergeruch, wobei die Vergleichsproben der Haut im axillaren Bereich entnommen werden,
b) Identifizierung von Keimen, die bei den Probanden mit starkem Körpergeruch im Vergleich zu den Probanden mit schwachem Körpergeruch vermehrt oder vermindert auftreten, durch Analyse der Zusammensetzungen der Vergleichsproben durch molekularbiologische Methoden, ausgewählt aus FISH, DGGE, TGGE, Real-Time-PCR, DNA-Arrays, SAGE oder DNA-Sequenziertechniken.

Ein weiterer Gegenstand der vorliegenden Erfindung ist daher ebenso ein Verfahren zur Ermittlung von in Bezug auf Körpergeruch präbiotisch wirksamen Substanzen, bestehend aus folgenden Schritten:
a) Entnahme von Vergleichsproben von Probanden mit starkem und schwachem Körpergeruch, wobei die Vergleichsproben der Haut im axillaren Bereich entnommen werden,
b) Identifizierung von Keimen, die bei den Probanden mit starkem Körpergeruch im Vergleich zu den Probanden mit schwachem Körpergeruch vermehrt oder vermindert auftreten, durch Analyse der Zusammensetzungen der Vergleichsproben durch molekularbiologische Methoden, ausgewählt aus FISH, DGGE, TGGE, Real-Time-PCR, DNA-Arrays, SAGE oder DNA-Sequenziertechniken,
c) Ermittlung von Substanzen, die das Wachstum und/oder die physiologische Aktivität und/oder die Überlebensfähigkeit der gemäß (b) vermehrt auftretenden Keime hemmen und/oder das Wachstum und/oder die physiologische Aktivität und/oder die Überlebensfähigkeit der gemäß (b) vermindert auftretenden Keime fördern.

Bei dem gemäß (b) identifizierten Geruchskeim handelt es sich in einer bevorzugten Ausführungsform um geruchsbildende Staphylokokken oder Anaerokokken.

Bei den gram-positiven anaeroben Kokken handelt es sich erfindungsgemäß bevorzugt um Bakterien der Gattung *Peptostreptococcus.* Die Gattungbezeichnung Peptostreptococcus beinhaltet die Gattungssynonyme *Peptoniphilus, Gallicola, Slackia, Anaerococcus (u.a. Anaerococcus octavius), Finegoldia, Micromonas, Atopobium* sowie *Ruminococcus.* Die an der Körpergeruchsbildung beteiligten Gram-positiven anaereoben Kokken, gegen die die erfindungsgemäßen ermittelten Substanzen wirksam sind, sind daher in einer bevorzugten Ausführungsform ausgewählt aus Bakterien dieser Gattungen.

Unter "geruchsbildenden Keimen" bzw. "Geruchskeimen" sind erfindungsgemäß grundsätzlich solche Mikroorganismen zu verstehen, die bei Menschen mit Körpergeruch vermehrt auftreten. Bevorzugt handelt es sich hierbei um Mikroorganismen, die entweder selbst Substanzen produzieren bzw. die Bildung von Substanzen fördern, die einen unangenehmen Geruch verursachen. Des weiteren kann es sich hierbei auch um Mikroorganismen handeln, die nur mittelbar in die Bildung solcher Substanzen involviert sind, beispielsweise dadurch, dass sie eine Substanz produzieren bzw. die Bildung von Substanzen fördern, die durch andere Mikroorganismen zu unangenehm riechenden Substanzen umgesetzt werden können. Die geruchsbildenden Mikroorganismen müssen also erfindungsgemäß nicht notwendigerweise selbst den unangenehmen Geruch verursachen, sondern können auch in anderer Weise in den Metabolismus der Geruchsbildung involviert sein.

In einer bevorzugten Ausführungsform handelt es sich bei den Substanzen, die gegen Körpergeruch wirksam sind, um präbiotisch wirksame Substanzen. Unter präbiotischer Wirkung ist erfindungsgemäß zu verstehen, dass das Wachstum und/oder die physiologische Aktivität und/oder die Überlebensfähigkeit der erwünschten Keime der Mikroflora eines bestimmten Habitats gegenüber dem Wachstum und/oder der physiologischen Aktivität und/oder der Überlebensfähigkeit der unerwünschten Keime der Mikroflora des betreffenden Habitats gefördert wird. Dies kann sowohl dadurch erreicht werden, dass der Wirkstoff förderlich auf das Wachstum der erwünschten Keime wirkt, ohne unmittelbar Einfluss auf das Wachstum der unerwünschten Keime zu haben, als auch dadurch, dass der Wirkstoff hemmend auf das Wachstum der unerwünschten Keime wirkt, ohne unmittelbar Einfluss auf das Wachstum der erwünschten Keime zu haben. In einer erfindungsgemäß besonders bevorzugten Ausführungsform jedoch wirkt der Wirkstoff förderlich auf das Wachstum der erwünschten Keime und wirkt zugleich hemmend auf das Wachstum der unerwünschten Keime. Erfindungsgemäß handelt es sich bei den erwünschten Keimen um geruchsneutrale Keime und bei den unerwünschten Keimen um geruchsbildende Keime. Bei dem Habitat handelt es sich vorzugsweise um die Haut, vor allem im axillaren Bereich.

Bei den Keimen, die gemäß (b) bei Probanden mit starkem Körpergeruch vermehrt auftreten, handelt es sich in einer bevorzugten Ausführungsform um geruchsbildende Staphylokokken oder um, insbesondere geruchsbildende, gram-positive anaerobe Kokken. Bei den Keimen, die gemäß (b) bei Probanden mit starkem Körpergeruch vermindert auftreten, handelt es sich in einer bevorzugten Ausführungsform um geruchsneutrale Staphylokokken. Gegenstand der vorliegenden Erfindung ist daher auch ein Verfahren zur Ermittlung von in Bezug auf Körpergeruch bzw. in Bezug auf die Körpergeruch verursachende Mikroflora präbiotisch wirksamer Substanzen, dadurch gekennzeichnet, dass Substanzen ermittelt werden, die das Wachstum, die physiologische Aktivität und/oder die Überlebensfähigkeit von gram-positiven anaeroben Kokken, insbesondere von *Anaerococcus octavius,* und/oder von geruchsbildenden Staphylokokken, insbesondere *Staphylococcus hominis,* hemmen, und/oder das Wachstum, die physiologische Aktivität und/oder die Überlebensfähigkeit von geruchsneutralen Staphylokokken, insbesondere *Staphylococcus epidermidis,* fördern, insbesondere durch Durchsuchen (Screenen) einer Substanzbibliothek.

Bei den erfindungsgemäß einsetzbaren molekularbiologischen Methoden gemäß Schritt (b), die insbesondere zur Evaluierung der Deoleistung in vivo und in vitro geeignet sind, handelt es sich um Fluoreszenz in situ Hybridisierung (FISH), Denaturierende Gradienten Gelelektorphorese (DGGE), Temperaturgradienten-Gelelektrophorese (TGGE), Real-Time PCR (Genome Res. (1996) 6(10) 986-94), DNA-Arrays (Applied an Environmental Micorbiology (2001) 67(8) 3677-82), SAGE (Velculescu,V.E. et al. (1995) Serial analysis of gene expression. Science, 270, 484-487) sowie um DNA-Sequenziertechniken (Olsen, G. 1988. Phylogenetic analysis using ribosomal RNA. Methods Enzymol. 164:793-812).

FISH steht hierbei für "Fluoreszenz-in-situ-Hybridisierung". Dieses Verfahren wurde zu Beginn der 90er Jahre entwickelt. Als Sonden werden fluoreszenzmarkierte Oligonukleotide eingesetzt. Das Verfahren ist bereits zur Untersuchung vieler Umweltproben erfolgreich zum Einsatz gekommen (Amann et al. (1990) J. Bacteriol. 172, 762). Das Verfahren macht sich den Umstand zunutze, dass die in jeder Zelle vorkommenden ribosomalen Ribonukleinsäuren (RNAs) sowohl hochkonservierte als auch variable, d.h. gattungs- oder sogar artspezifische Sequenzen aufweisen. Gegen diese Sequenzbereiche können komplementäre Oligonukleotide hergestellt werden, die zusätzlich mit einem detektierbaren Marker versehen werden können. Mittels dieser sogenannten Nukleinsäuresonden können Mikroorganismenarten, -gattungen oder -gruppen hochspezifisch direkt in der Probe identifiziert und bei Bedarf auch visualisiert oder quantifiziert werden. Auf diese Weise ist eine verzerrungsfreie Darstellung der tatsächlichen in situ-Verhältnisse der Biozönose möglich. Sogar bisher nicht-kultivierte und demnach nicht beschriebene Mikroorganismen können identifiziert werden. Bei der FISH-Methode dringen Sonden in die in der untersuchten Probe vorhandenen Zellen ein. Sofern ein Mikroorganismus der Art, Gattung oder Gruppe, für welche die Sonden entwickelt wurden, in der untersuchten Probe vorhanden ist, binden die Sonden in der Mikroorganismenzelle an ihre Zielsequenz, und die Zellen können aufgrund der Markierung der Sonden detektiert werden.

DGGE steht für "denaturierende Gradienten-Gelelektrophorese". Es handelt sich hierbei um ein Trennverfahren, dass sich Unterschied im Schmelzverhalten unterschiedlicher doppelsträngiger DNA-Fragmente zunutze macht. Ursprünglich wurde diese Methode entwickelt um Punktmutationen gelelektrophoretisch nachzuweisen, so dass es sich hinsichtlich der Auflösung um eine sehr empfindliche Methode handelt (Fisher und Lerman (1979) Cell 16, 191-200; Myers et al. (1987) Methods in Enzymology 212, 71-104; Electrophoresis (1989) 10(5-6)377-89). DGGE wird in jüngerer Zeit sehr häufig zur Untersuchung natürlicher Habitate eingesetzt (Muyzer et al. (1996) Molecular Microbial Ecology Manual 3.4.4:1-23: Eds: Akkermans et al., Holland 1996; Diez et al. (2001) Appl. Environ. Microbiol. 67(7), 2942-2951).

TGGE steht für "Temperatur-Gradienten-Gelelektrophorese". Im Gegensatz zur DGGE wird statt mit einem denaturierenden Agens mit einem Temperaturgradienten gearbeitet. Die Methoden sind ansonsten äquivalent (Riesner,D. et al. (1989) Electrophoresis 10, 377-389).

Sobald die für die Geruchsbildung bzw. Geruchshemmung verantwortlichen Keime ermittelt worden sind, kann die Suche nach, gegebenenfalls selektiv, präbiotisch wirksamen Substanzen gemäß Schritt (c) auf unterschiedliche Art und Weise erfolgen. Eine den zu untersuchenden Mikroorganismen enthaltenden Probe kann hierzu mindestens einer auf ihre präbiotische Wirksamkeit hin zu untersuchenden Substanz ausgesetzt werden, wobei als Vergleichsprobe vorzugsweise eine Probe dient, die die zu untersuchende Substanz nicht enthält, ansonsten aber gleich zusammengesetzt ist. Durch Ermittlung von Unterschieden im Erscheinungsbild (Phänotyp), der physiologischen Aktivität und/oder in der Anzahl, insbesondere Konzentration, des mindestens einen zu untersuchenden Mikroorganismus zwischen Probe und Vergleichsprobe kann nun ermittelt werden, ob ein präbiotischer Effekt vorliegt.

Bei dem mindestens einen bei Körpergeruch vermehrt auftretenden Mikroorganismus handelt es sich in einer bevorzugten Ausführungsform um gram-positive anaerobe Kokken, insbesondere um Peptostreptokokken, vor allem um *Anaerococcus octavius,* um geruchsbildende Staphylokokken, insbesondere um *Staphylococcus hominis,* um geruchsbildende Corynebakterien, insbesondere *Corynebakterium CDC G2,* und/oder um *Micrococcus luteus.* Bei dem mindestens einen bei Körpergeruch vermindert auftretenden Mikroorganismus handelt es sich in einer bevorzugten Ausführungsform um geruchsneutrale Staphylokokken, insbesondere um *Staphylococcus epidermidis.*

Bei dem Verfahren handelt es sich vorzugsweise um ein in-vitro-Verfahren. So kann auf einfache Weise eine Vielzahl von Stoffen darauf hin untersucht werden, ob sie einen Effekt auf eine Vielzahl unabhängig voneinander untersuchbarer Mikroorganismen haben. Auf diese Weise können auf einfache Weise Stoffe, die eine potentiell präbiotische Wirkung besitzen, ausfindig gemacht werden.

In einer bevorzugten Ausführungsform enthält die Mikroorganismen enthaltende Probe hierbei keinen anderen Organismus als den auf seine Beeinflussung durch die zugegebene Substanz hin zu untersuchenden. Dies wird dadurch erreicht, dass gezielt dieser Organismus, vorzugsweise in Flüssigkultur (im Schüttelkolben, in Reaktionsgefäßen oder in Zellkulturplatten) oder auf Agarplatten kultiviert wird. Dies hat den Vorteil, dass unmittelbar die Vermehrung des Mikroorganismus als Indikator für die Beeinflussung durch die zu untersuchende Substanz verwendet werden kann, die unmittelbar optisch, insbesondere mikroskopisch, durch Ausplattieren auf Agarplatten oder durch eine einfache technische Methode, wie beispielsweise spektrometrische Bestimmung der optischen Dichte, bestimmt werden kann. Auf einen hohen technischen und zeitlichen Aufwand kann hierbei verzichtet werden.

In einer bevorzugten Ausführungsform wird hierbei durch Kultivierung zunächst überprüft, ob eine auf ihre präbiotische Wirksamkeit hin zu untersuchende Substanz einen positiven Effekt auf einen erwünschten Mikroorganismus hat. In einem zweiten Schritt wird dann untersucht, welchen Effekt die als positiv auf den erwünschten Mikroorganismus ausfindig gemachte Substanz auf unerwünschte Mikroorganismen (bzw. gegebenenfalls auf andere erwünschte Mikroorganismen) hat.

Umgekehrt kann auch zunächst überprüft werden, ob eine auf ihre präbiotische Wirksamkeit hin zu untersuchende Substanz einen erwünschten, also negativen, Effekt auf einen unerwünschten Mikroorganismus hat. Anschließend kann dann untersucht werden, welchen Effekt die als negativ auf den unerwünschten Mikroorganismus einwirkende Substanz auf erwünschte Mikroorganismen (bzw. gegebenenfalls auch auf andere unerwünschte Mikroorganismen) hat.

In einer anderen bevorzugten Ausführungsform kann die Mikroorganismen enthaltende Probe auch mehrere Organismen enthalten. Es kann sich hierbei beispielsweise um ein Kollektiv erwünschter bzw. geruchsneutraler Mikroorganismen eines bestimmten Habitats oder aber um ein Kollektiv unerwünschter bzw. geruchsbildender Mikroorganismen eines bestimmten Habitats handeln. Es kann sich hierbei aber auch um eine Probe handeln, die einem natürlichen Habitat entnommen und anschließend, vorzugsweise in Schüttelkolben, auf Agarplatten oder in Zellkultur, kultiviert wurde.

Alternativ kann auch direkt eine Probe, die einem natürlichen Habitat entnommen wurde, also eine in-vivo-Probe, analysiert werden, ohne dass diese zuvor kultiviert wurde. Als Vergleichsprobe wird in diesem Fall entsprechend eine in-vivo-Probe verwendet, die demselben Habitat entstammt und mindestens einer auf ihre präbiotische Wirksamkeit hin zu untersuchenden Substanz ausgesetzt wurde.

Im Falle der Kultivierung der Mikroorganismen enthaltenden Probe in vitro wird die Vergleichsprobe dadurch erhalten, dass eine Kultivierung unter den selben Bedingungen erfolgt wie Kultivierung der Probe, die die mindestens eine auf ihre präbiotische Wirkung hin zu untersuchende Substanz enthält, mit dem Unterschied, dass keine Inkubation mit der mindestens einen zu untersuchenden Substanz erfolgt. Hierzu kann eine Kultur nach der Animpfung in zwei gleich große Volumina aufgeteilt werden und eine von den beiden so erhaltenen Kulturen mit der mindestens einen auf ihre präbiotische Wirkung hin zu untersuchende Substanz angeimpft werden, während die andere Probe als Vergleichsprobe dient. Alternativ können beispielsweise auch zwei gleich große Medienvolumina mit der gleichen Menge an Mikroorganismen enthaltenden Probe angeimpft und unter denselben Bedingungen kultiviert werden, wobei die eine Kultur der mindestens einen auf ihre präbiotische Wirkung hin zu untersuchenden Substanz ausgesetzt wird und die andere nicht.

In einer bevorzugten Ausführungsform des Verfahrens erfolgt Inkubation mit genau einer auf ihre präbiotische Wirkung hin zu untersuchenden Substanz. Dies hat den Vorteil, dass direkt ersichtlich wird, ob diese Substanz einen Einfluss auf das Wachstum, die physiologische Aktivität bzw. die Überlebensfähigkeit des mindestens einen Mikroorganismus der Mikroorganismen enthaltenden Probe hat.

In einer anderen bevorzugten Ausführungsform erfolgt Inkubation mit einer Mischung aus mehreren auf ihre präbiotische Wirkung hin zu untersuchenden Substanzen. Dieses Verfahren ist insbesondere dann anwendbar, wenn davon ausgegangen wird, dass die Anzahl der Treffer, also die Anzahl der als wirksam identifizierbaren Substanzen, klein sein wird. Es kann dann zunächst ein Vorscreening erfolgen, um zu ermitteln, ob die Mischung Substanzen enthält, die präbiotische Wirkung zeigen. In einem zweiten Schritt kann dann durch ein Einzel-Screening untersucht werden, auf welche Substanzen der nachgewiesene Effekt zurückzuführen ist.

Bei dem Habitat handelt es sich um die Haut, vor allem die menschliche Haut, im axillaren Bereich. Erfindungsgemäß sind unter dem Begriff "Haut" bevorzugt die Haut selbst, insbesondere die menschliche Haut, daneben aber auch Hautanhangsgebilde, sofern sie lebende Zellen umfassen, insbesondere Haarfollikel, Haarwurzel, Haarzwiebel, sowie Talgdrüsen und Schweißdrüsen zu verstehen. Die Untersuchungen werden vorzugsweise geschlechtsspezifisch durchgeführt.

Grundsätzlich kann es sich bei dem mindestens einen zu untersuchenden Mikroorganismus um jeden beliebigen Mikroorganismus handeln, der für seine Involvierung in die Geruchsbildung bekannt ist, sei es dass er selbst zur Geruchsbildung beiträgt oder dass er dazu in der Lage ist geruchsbildende Mikroorganismen zu verdrängen. In einer bevorzugten Ausführungsform handelt es sich bei dem Mikroorganismus jedoch um einen Mikroorganismus, der aufgrund eines der zuvor beschriebenen erfindungsgemäßen Verfahren als potentiell geruchsbildend und/oder geruchshemmend identifiziert worden ist.

Bei der Mikroorganismen enthaltenden Probe handelt es sich in einer bevorzugten Ausführungsform um eine Probe, die der Haut, insbesondere der menschlichen Haut, im axillaren Bereich, entnommen wurde und anschließend gegebenenfalls in vitro kultiviert wurde, wobei das betreffende Habitat, der die Probe entnommen wurde, den mindestens einen zu untersuchenden Mikroorganismus natürlicherweise enthält oder aufgrund einer pathogenen Veränderung enthalten kann. Andererseits kann die Mikroorganismen enthaltenden Probe auch als einzigen Mikroorganismus den mindestens einen zu untersuchenden Mikroorganismus oder gegebenenfalls weitere Mikroorganismen, die vorzugsweise gezielt mit dem mindestens einen zu untersuchenden Mikroorganismus vorzugsweise in vitro kultiviert wurden, enthalten. In einer besonders bevorzugten Ausführungsform handelt es sich bei dem Habitat um den menschlichen, insbesondere männlichen, Achselbereich und der Mikroorganismus tritt vorzugsweise vermehrt bei Körpergeruch auf.

Die Analyse des Effektes der mindestens einen auf ihre präbiotische Aktivität hin zu untersuchenden Substanz kann erfindungsgemäß außer durch die Methoden Spektrometrie, Mikroskopie und Ausplattieren beispielsweise auch durch die bereits zuvor genannten molekularbiologischen Methoden, insbesondere FISH, DGGE, TGGE (Electrophoresis (1989) 10(5-6), 377-89), RT-PCR (Real time-PCR) (Genome Res. (1996) 6(10), 986-94) oder DNA-Microarrays (Applied and Environmental Microbiology (2001) 67(8), 3677-82), oder durch Antikörper-Assays, insbesondere ELISA, erfolgen. Diese Verfahren sind vorteilhafterweise vor allem dann anwendbar, wenn die Mikroorganismen enthaltende Probe unterschiedliche Mikroorganismen enthält, so dass ein rein quantitatives Verfahren, das eine Differenzierung zwischen den Mikroorganismen nicht erlaubt, wie etwa die spektrometrische Bestimmung der optischen Dichte, nicht geeignet ist, den Effekt der zu untersuchenden Substanz differenziert nach den unterschiedlichen Mikroorganismen wiederzugeben.

Des weiteren wird beschrieben, daβ die präbiotisch wirksamen Substanzen, die mit Hilfe eines der zuvor genannten erfindungsgemäßen Verfahren erhältlich sind, zur Herstellung von Kosmetika oder Arzneimittel, vor allem zur Hemmung des Wachstums geruchsbildender Keime und/oder zur Förderung des Wachstums geruchsneutraler Keime, insbesondere im axillaren Bereich, geeignet sind.

Bei diesen Arzneimitteln und Kosmetika handelt es sich um solche zur topischen Applikation, insbesondere um Desodorantien und/oder Antitranspirantien. Diese können entsprechend weitere Komponenten enthalten wie sie für solche Mittel üblich sind, insbesondere ausgewählt aus der Gruppe bestehend aus Vitaminen, Provitaminen oder Vitaminvorstufen der Vitamin B-Gruppe oder deren Derivate sowie Derivate von 2-Furanon, Panthenol, Pantolacton, Nicotinsäureamid und Biotin, Pflanzenextrakte, MMP-1-inhibierende Substanzen, Ester von Retinol (Vitamin A₁) mit einer C₂₋₁₈-Carbonsäure, oberflächenaktive Substanzen als Emulgator oder Dispergiermittel, Aminosäuren und deren Zinksalze und deren Säureadditionssalze, filmbildende und/oder emulsionsstabilisierende und/oder verdickende und/oder adhäsive Polymere, Fettstoffe, Tenside, Anti-Transpirantien und Polyole, organische, mineralische und modifizierte mineralische Lichtschutzfilter, Proteinhydrolysate und deren Derivate, Mono-, Oligo- und Polysaccharide sowie deren Derivate, α-Hydroxycarbonsäuren und α-Ketocarbonsäuren sowie deren Ester-, Lacton- oder Salzformen.

## Patentansprüche

1. Verfahren zur Ermittlung von Keimen, die für die Geruchsbildung und/oder Geruchshemmung verantwortlich sind, bestehend aus folgenden Schritten:
a) Entnahme von Vergleichsproben von Probanden mit starkem und schwachem Körpergeruch, wobei die Vergleichsproben der Haut im axillaren Bereich entnommen werden,
b) Identifizierung von Keimen, die bei den Probanden mit starkem Körpergeruch im Vergleich zu den Probanden mit schwachem Körpergeruch vermehrt oder vermindert auftreten, durch Analyse der Zusammensetzungen der Vergleichsproben durch molekularbiologische Methoden ausgewählt aus FISH, DGGE, TGGE, Real-Time-PCR, DNA-Arrays, SAGE oder DNA-Sequenziertechniken.

2. Verfahren zur Ermittlung von in Bezug auf Körpergeruch präbiotisch wirksamen Substanzen, bestehend aus folgenden Schritten:
a) Entnahme von Vergleichsproben von Probanden mit starkem und schwachem Körpergeruch, wobei die Vergleichsproben der Haut im axillaren Bereich entnommen werden,
b) Identifizierung von Keimen, die bei den Probanden mit starkem Körpergeruch im Vergleich zu den Probanden mit schwachem Körpergeruch vermehrt oder vermindert auftreten, durch Analyse der Zusammensetzungen der Vergleichsproben durch molekularbiologische Methoden ausgewählt aus FISH, DGGE, TGGE, Real-Time-PCR, DNA-Arrays, SAGE oder DNA-Seq uenziertechniken,
c) Ermittlung von Substanzen, die das Wachstum und/oder die physiologische Aktivität und/oder die Überlebensfähigkeit der gemäß (b) vermehrt auftretenden Keime hemmen und/oder das Wachstum und/oder die physiologische Aktivität und/oder die Überlebensfähigkeit der gemäß (b) vermindert auftretenden Keime fördern.

## Claims

1. Method for determining germs that are responsible for the formation of odour and/or of odour inhibition, consisting of the following steps:
a) sampling comparative samples from subjects with a strong and slight body odour, wherein the comparative samples are taken from the skin in the axillary region of the skin,
b) identifying germs that occur to a greater or lesser extent in the subjects with a strong body odour in comparison to the subjects with a slight body odour by analysing the compositions of the comparative samples by means of molecular biological methods selected from FISH, DGGE, TGGE, Real-Time-PCR, DNA-Arrays, SAGE or DNA-sequencing techniques.

2. Method for determining substances with a prebiotic activity in relation to body odour, consisting of the following steps:
a) sampling comparative samples from subjects with a strong and slight body odour, wherein the comparative samples are taken from the skin in the axillary region of the skin,
b) identifying germs that occur to a greater or lesser extent in the subjects with a strong body odour in comparison to the subjects with a slight body odour by analysing the compositions of the comparative samples by means of molecular biological methods selected from FISH, DGGE, TGGE, Real-Time-PCR, DNA-Arrays, SAGE or DNA-sequencing techniques,
c) determining, substances that inhibit the growth and/or the physiological activity and/or the survivability of the increasingly occurring germs according to (b) and/or that promote the growth and/or the physiological activity and/or the survivability of the decreasingly occurring germs according to (b).

## Revendications

1. Procédé pour la détermination de germes qui sont responsables de l'apparition et/ou de l'inhibition d'odeurs, constitué par les étapes suivantes :
a) prélèvements d'échantillons comparatifs sur des sujets présentant une forte et une faible odeur corporelle, les échantillons comparatifs étant prélevés sur la peau dans la zone axillaire,
b) identification de germes qui apparaissent de manière augmentée ou réduite chez les sujets présentant une forte odeur corporelle par rapport aux sujets présentant une faible odeur corporelle, par analyse des compositions des échantillons comparatifs par des procédés de biologie moléculaire choisis parmi les procédés FISH, DGGE, TGGE, PCR en temps réel, puces à ADN, SAGE ou les techniques de séquençage d'ADN.

2. Procédé pour la détermination de substances à action prébiotique par rapport à l'odeur corporelle, constitué par les étapes suivantes :
a) prélèvements d'échantillons comparatifs sur des sujets présentant une forte et une faible odeur corporelle, les échantillons comparatifs étant prélevés sur la peau dans la zone axillaire,
b) identification de germes qui apparaissent de manière augmentée ou réduite chez les sujets présentant une forte odeur corporelle par rapport aux sujets présentant une faible odeur corporelle, par analyse des compositions des échantillons comparatifs par des procédés de biologie moléculaire choisis parmi les procédés FISH, DGGE, TGGE, PCR en temps réel, puces à ADN, SAGE ou les techniques de séquençage d'ADN,
c) détermination de substances qui inhibent la croissance et/ou l'activité physiologique et/ou l'aptitude à la survie des germes qui apparaissent de manière augmentée selon (b) et/ou qui favorisent la croissance et/ou l'activité physiologique et/ou l'aptitude à la survie des germes qui apparaissent de manière réduite selon (b).
